# EUROPEAN PATENT SPECIFICATION

(11) **EP 3 329 005 B1**
(45) Date of publication and mention of the grant of the patent: **13.05.2020**
(21) Application number: 16831427.6
(22) Date of filing: 29.07.2016
(51) Int. Cl.: C12Q 1/6883

(54) **MICRORNA BIOMARKERS FOR TRAUMATIC BRAIN INJURY AND METHODS OF USE THEREOF**
MIKRORNA-BIOMARKER FÜR HIRNTRAUMA UND VERFAHREN ZUR VERWENDUNG DAVON
BIOMARQUEURS DE MICRO-ARN UTILES POUR UNE LÉSION CÉRÉBRALE TRAUMATIQUE ET PROCÉDÉS D'UTILISATION DE CEUX-CI

(30) Priority: 29.07.2015 US 201562198295 P
(43) Date of publication of application: 06.06.2018
(73) Proprietor: The Henry M. Jackson Foundation for the Advancement of Military Medicine, Inc., Bethesda, MD 20817 (US); University of Florida Research Foundation, Inc., Gainesville, FL 32611 (US); Orlando Health, Inc. d/b/a Orlando Regional Medical Center, Orlando, FL 32806 (US)
(72) Inventor: MAHESHWARI, Radha K., Rockville, Maryland 20850 (US); BALAKATHIRESAN, Nagaraja S., Clarksburg, Maryland 20871 (US); BHOMIA, Manish, Rockville, Maryland 20851 (US); WANG, Kevin K., Gainesville, Florida 32607 (US); PAPA, Linda, Windermere, Florida 34786 (US)
(74) Representative: Gevers & Orès
(86) International application number: PCT/US2016/044784
(87) International publication number: WO 2017/019976

(56) References cited:
- WO-A1-2015/196191
- US-A1- 2007 092 882
- US-A1- 2009 137 505
- US-A1- 2013 022 982
- US-A1- 2013 022 982
- US-A1- 2015 119 278
- JOHN B. REDELL ET AL: "Human Traumatic Brain Injury Alters Plasma microRNA Levels", JOURNAL OF NEUROTRAUMA., vol. 27, no. 12, 1 December 2010 (2010-12-01), pages 2147-2156, XP055353337, US ISSN: 0897-7151, DOI: 10.1089/neu.2010.1481
- YI ZHANG ET AL: "Altered expression levels of miRNAs in serum as sensitive biomarkers for early diagnosis of traumatic injury", JOURNAL OF CELLULAR BIOCHEMISTRY, vol. 112, no. 9, 1 September 2011 (2011-09-01), pages 2435-2442, XP055530556, ISSN: 0730-2312, DOI: 10.1002/jcb.23168
- MANISH BHOMIA ET AL: "A Panel of Serum MiRNA Biomarkers for the Diagnosis of Severe to Mild Traumatic Brain Injury in Humans", SCIENTIFIC REPORTS, vol. 6, no. 1, 24 June 2016 (2016-06-24), XP055356432, DOI: 10.1038/srep28148
- NAGARAJA BALAKATHIRESAN ET AL: "MicroRNA Let-7i Is a Promising Serum Biomarker for Blast-Induced Traumatic Brain Injury", JOURNAL OF NEUROTRAUMA., vol. 29, no. 7, 1 May 2012 (2012-05-01), pages 1379-1387, XP055356447, US ISSN: 0897-7151, DOI: 10.1089/neu.2011.2146
- ANUJ SHARMA ET AL: "Identification of Serum MicroRNA Signatures for Diagnosis of Mild Traumatic Brain Injury in a Closed Head Injury Model", PLOS ONE, vol. 9, no. 11, 7 November 2014 (2014-11-07), page e112019, XP055357235, DOI: 10.1371/journal.pone.0112019
- BALAKATHIRESAN NAGARAJA S ET AL: "Serum and amygdala microRNA signatures of posttraumatic stress: Fear correlation and biomarker potential", JOURNAL OF PSYCHIATRIC RESEARCH, ELSEVIER LTD, GB, vol. 57, 21 June 2014 (2014-06-21), pages 65-73, XP029017961, ISSN: 0022-3956, DOI: 10.1016/J.JPSYCHIRES.2014.05.020
- BHOMIA ET AL.: 'A Panel of Serum MiRNA Biomarkers for the Diagnosis of Severe to Mild Traumatic Brain Injury in Humans' SCIENTIFIC REPORTS vol. 6, no. 1, 24 June 2016, pages 1 - 12, XP055356432
- CHANDRAN ET AL.: 'Differential expression of microRNAs in the brains of mice subjected to increasing grade of mild traumatic brain injury' BRAIN INJURY vol. 31, no. 1, 07 November 2016, pages 106 - 119, XP055372653

## Description

### Field of Invention

The present invention relates to methods of diagnosing traumatic brain injury (TBI) in a subject.

### Background of the Invention

Traumatic brain injury (TBI) is a problem with epidemic magnitude involving both civilian, military service members and professional athletes. In the United States, more than 1.3 million emergency room visits account for TBI and is a cause of almost a third of all injury related deaths. The economic burden of TBI in the United States is estimated to be $76.5 billion annually, in total lifetime direct medical costs and productivity losses.

Mild TBI (mTBI), also called concussion, accounts for more than 77 % of the total reported TBI cases in the United States. Among these cases it is estimated that around 40% of injuries are often ignored and do not seek medical attention. mTBI is also a major cause of morbidity in the veterans returning from the recent wars with more than 20% of the veterans returning from the recent wars in Iraq and Afghanistan experienced a mTBI. Most of the symptoms associated with mTBI resolve within days or weeks of injury with substantial recovery in most cases. However, approximately, 10-20% of mTBI patients complain of prolonged problems and some experience symptoms lasting more than a year. mTBI can induce neurological, cognitive and behavioral changes in an individual. The clinical symptoms may include headaches, sleep disturbance, impaired memory, anxiety and depression. The accelerating and decelerating forces during the impact to the head also results in the injury to the white matter causing diffuse axonal injury. Axonal injury may peak at 24 h post injury and can progress up to a year post injury. It is believed that this continuous progression may be a causative factor for the poor outcome post mTBI.

mTBI usually is a challenge for the clinicians to diagnose because of the lack of apparent signs of a brain injury. mTBI is currently assessed using the Glasgow comma scale (GCS) which measure a score by assessing the eye, verbal, and motor response of the patient. GCS score and loss or alterations of consciousness are used to determine the severity of the injury. The GCS score can be of limited use in mTBI diagnosis due to the presence of polytrauma, alcohol abuse, use of sedatives and psychological stress. Computed tomography (CT) and magnetic resonance imaging (MRI) are used to detect the extent of brain injury, however, in case of a concussion, CT and MRIs often fail to detect any specific injury lesion due to limited sensitivity and absence of micro-bleeds. With new technological advancements, MRIs have become more sensitive than CT but due to their limited availability and the cost of the scan makes the utilization of this technique difficult for the acute stage diagnosis for both military and civilians.

Biomarkers in biofluids offer many advantages for mTBI diagnosis since they can be measured from the peripheral tissues such as blood, urine and saliva and can be easily quantitated using existing methods. Several protein markers in serum and cerebrospinal fluid (CSF) like S-100 calcium binding protein (S-100β), glial fibrillary acidic protein (GFAP) and Ubiquitin C-Terminal Hydrolase-L1 (UCH-L1) have been extensively studied for their utility as biomarkers for mild to severe TBI (sTBI). However, most of the protein biomarkers studied have relatively less sensitivity for mTBI with no intracranial lesions. Combinations of more than one protein biomarkers for mTBI diagnosis have been recently studied, and these show better diagnostic accuracy in comparison to single markers. Despite extensive studies most of the protein markers are in preclinical testing and none of the markers are available for clinical use.

MicroRNAs (miRNA) are small (19-28nt) endogenous RNA molecules that regulate protein synthesis at post transcriptional level. MiRNAs can be detected in serum and can be an indicator of disease pathology in the cell of origin including neuronal cells. This property of reflecting a diseased condition has recently gained attention towards miRNAs as biomarkers of central nervous system (CNS) pathology. Serum miRNAs are relatively stable and are resistant to repeated freeze thaw, enzymatic degradation and can survive variable pH conditions which make them a suitable biomarker candidate for mTBI.

MiRNAs have been recently reported as specific and sensitive biomarkers of many CNS diseases. The serum expression of miRNAs in response to a concussive mild injury in a closed head injury model was recently reported, and a signature of nine miRNAs was found to be modulated in serum immediately after the injury. MiRNA modulation was also analyzed in a rodent model of traumatic stress, and a signature of 9 miRNAs was identified which were upregulated in serum and amygdala of the animals 2 weeks post exposure to traumatic stress. Interestingly, miRNAs reported in this study did not have any similarities with the miRNAs reported for TBI studies, suggesting miRNA expression in serum may be a specific indicator of the altered physical state of the brain. US 2013/0022982 and Redell et al. (J Neurotrauma 2010, 27:2147-2156) disclose that human traumatic brain injury alters plasma microRNA levels. There remains a need for a non-invasive, sensitive reliable test for diagnosis and monitoring TBI.

### Summary of the Invention

The present invention is defined by the appended claims and relates to a method of diagnosing severe traumatic brain injury (TBI) in a human subject with a head injury, the method comprising: a) determining level(s) of one or more micro RNAs (miRNAs) in a serum biological sample taken from the subject; and b) comparing the determined level(s) of the one or more miRNAs against level(s) of the same one or more miRNAs from a control subject determined not to be suffering from TBI or from the same subject at an earlier timepoint; wherein the one or more miRNAs comprise hsa-miR-151-5p; and wherein a fold change of a level of hsa-mir-151-5p above 5 compared to the control level determined in step b) is indicative that the subject is suffering from severe TBI. Further disclosed herein are methods of diagnosing traumatic brain injury (TBI) in a subject, the method comprising (a) determining a level(s) of one or more specific microRNAs (miRNAs) in a biological sample taken from the subject, and (b) comparing the determined level(s) of the one or more miRNAs against a level(s) of the same one or more miRNAs from a control subject determined not to be suffering from TBI, wherein an increase in the level(s) of the one or more miRNAs compared to level(s) of the one or more miRNAs from the control subject determined not to be suffering from TBI is indicative that the subject may be suffering from TBI.

Further disclosed herein are methods of monitoring the progression of traumatic brain injury (TBI) in a subject, the method comprising (a) analyzing at least two biological samples from the subject taken at different time points to determine a level(s) of one or more specific miRNAs, and (b) comparing the level(s) of the one or more specific miRNAs over time to determine if the subject's level(s) of the one or more specific miRNAs is changing over time, wherein an increase in the level(s) of the one or more specific miRNAs over time is indicative that the subject's risk of suffering from TBI is increasing over time.

Further disclosed herein are methods of detecting a miRNA or plurality of microRNA's in a biological sample, comprising: obtaining a first biological sample from a subject presenting with clinical symptoms of a TBI; contacting said first biological sample with a probe for binding at least one miRNA; and detecting with Northern blot or a real-time PCR the presence or absence of the microRNA-cDNA complex, wherein the absence of the complex is indicative of the absence of the microRNA in the first biological sample.

In one aspect, said miRNA is selected from the group consisting of hsa-miR-328, hsa-miR-362-3p, hsa-miR-486, hsa-miR-151-5p, hsa-miR-942, hsa-miR-194, hsa-miR-361, hsa-miR-625*, hsa-miR-1255B, hsa-miR-381, hsa-miR-425*, hsa-miR-638, hsa-miR-93, hsa-miR-1291, hsa-miR-19a, hsa-miR-601, hsa-miR-660, hsa-miR-9*, hsa-miR-130b, hsa-miR-339-3p, hsa-miR-34a, hsa-miR-455, hsa-miR-579, hsa-miR-624, mmu-miR-491, hsa-miR-195, hsa-miR-30d, hsa-miR-20a, hsa-miR-505*, mmu-miR-451, hsa-miR-199a-3p, hsa-miR-27a, hsa-miR-27b, hsa-miR-296, hsa-miR-92a and hsa-miR-29c. In some embodiments, said miRNAs exclude one, two, three, four, five, six, seven, eight or more, or all of hsa-miR-425*, hsa-miR-942, hsa-miR-361, hsa-miR-93, hsa-miR-34a, hsa-miR-455, hsa-miR-624, mmu-miR-491, and hsa-miR-27a.

In another aspect, the TBI is mild TBI (mTBI) or severe TBI (sTBI). In another aspect, the TBI is a closed head injury (CHI) or a blast-induced traumatic brain injury (bTBI). In another aspect, the subject is human. In another aspect, the biological sample is a serum and/or plasma sample. In another aspect, the biological sample is taken from the subject less than one day, or less than 2, 3, 4, 5, 6, 7, 8, 9, 10, 11, 12, 13, or 14 days after the suspected traumatic episode.

In another aspect, the level(s) of one or more specific miRNAs are determined by a real time PCR. The methods of diagnosing the TBI according to some embodiments of the present specification further comprise amplifying the miRNAs.

### Brief Description of the Drawings

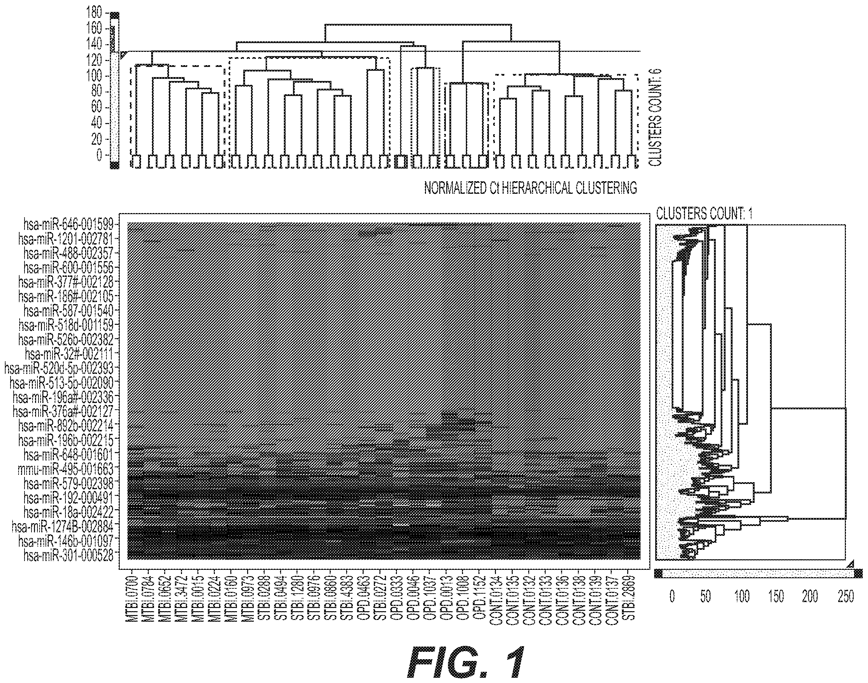

Figure 1 shows the hierarchical clustering of the miRNA profile for most miRNAs of all the samples using their delta Ct values to understand pattern of expression in different experimental groups. Control groups and the TBI groups and showed distinct changes. Orthopedic injury groups were distinct from control but most of these samples were clustered separately from the TBI groups.

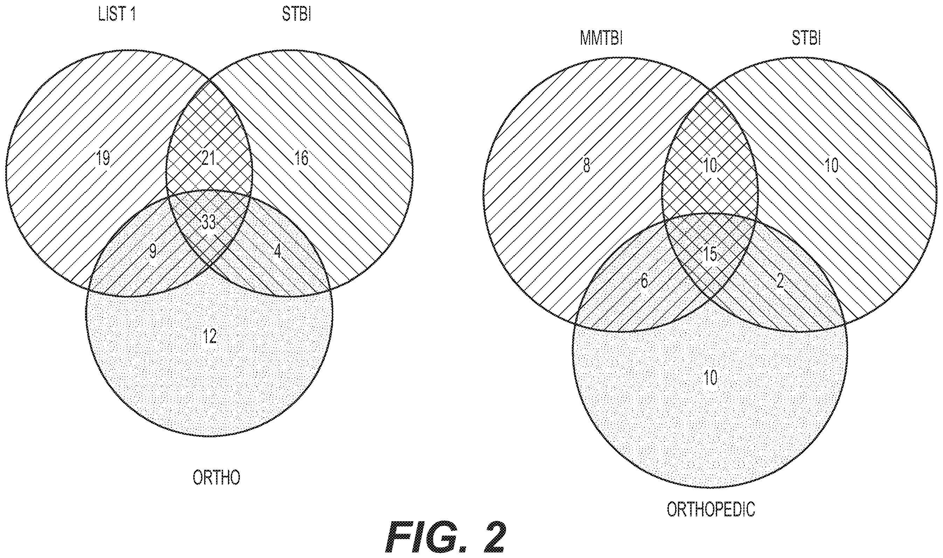

Figure 2 shows two exemplary Venn diagrams showing significant expression of miRNAs in mTBI, sTBI and orthopedic injury control groups over control samples in some embodiments. MiRNA expression was normalized using global normalization algorithm. Each of the injury group was normalized with the control samples to identify significantly modulated miRNAs in injury groups.

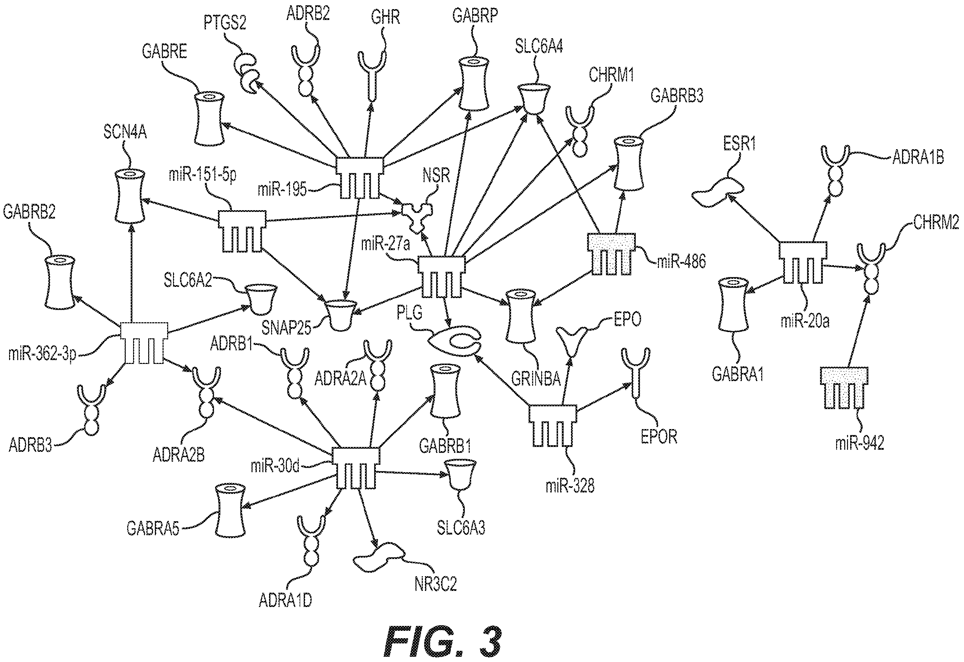

Figure 3 shows the ingenuity pathway analysis program that identifies direct targets for TBI miRNA candidates; hsa-miR-328, hsa-miR-362-3p, and hsa-miR-486 show upregulated expression.

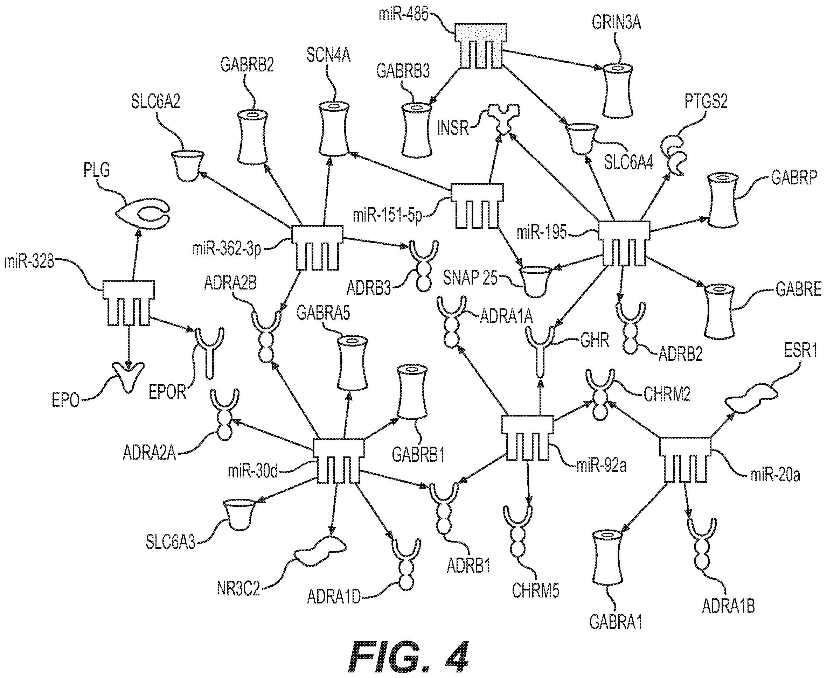

Figure 4 depicts another ingenuity pathway analysis program that identified direct targets for TBI miRNA candidates.

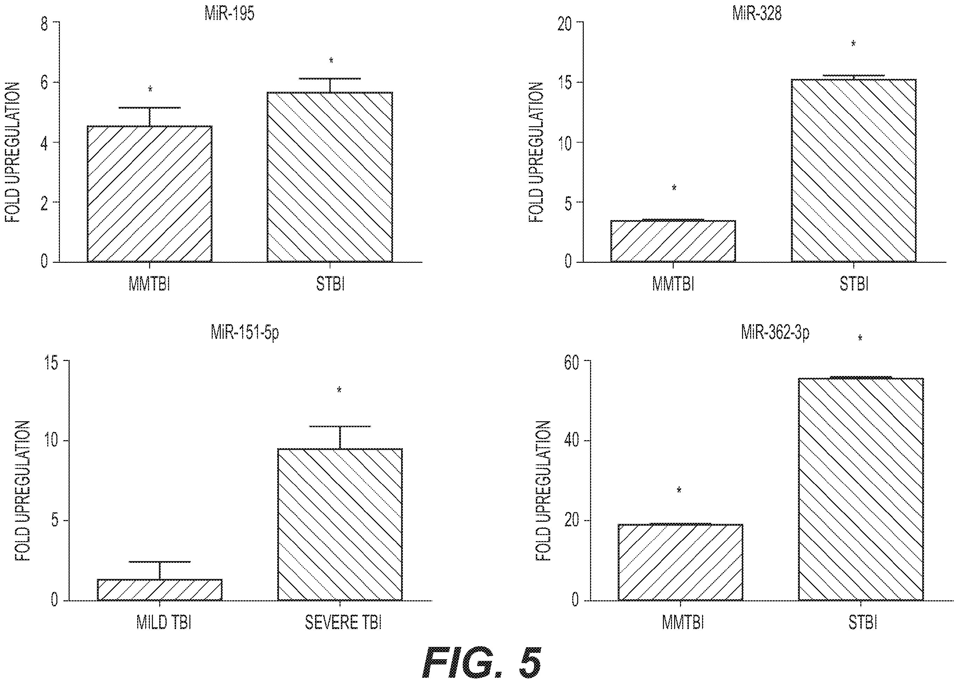

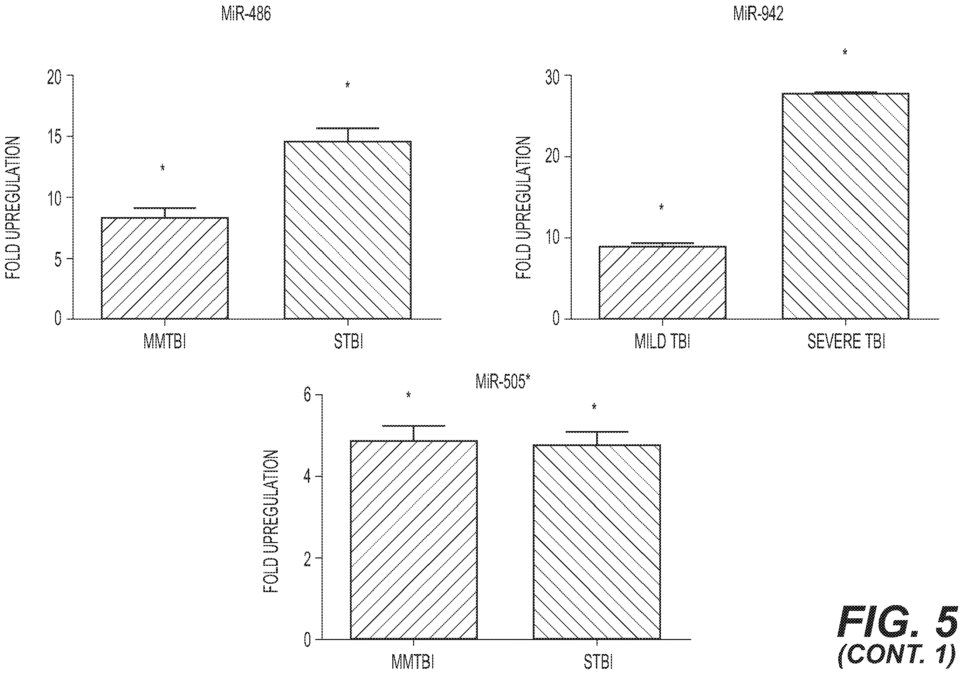

Figure 5 shows MiRNA specific validation assays in serum samples of mTBI and sTBI. Values are expressed as fold change ± SD over control in linear scale. Significance was calculated using paired student t test (*p*<*0.05*).

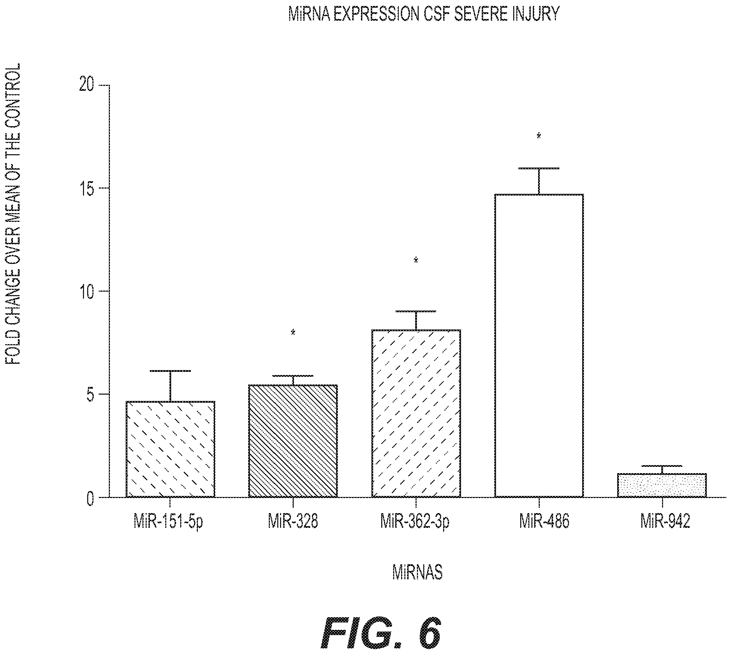

Figure 6 depicts miRNA specific validation assays in CSF samples of sTBI. Specific miRNA assays were performed for the five candidate miRNAs. Normalization was done with miR-202 which showed the least standard deviation and was selected as a normalizing control. Among the five tested, miRNAs, miR-328, miR-362-3p and miR-486 were significantly upregulated. Values are expressed as fold change ± SD over control in linear scale. Significance was calculated using paired student t test (*p*<*0.05*).

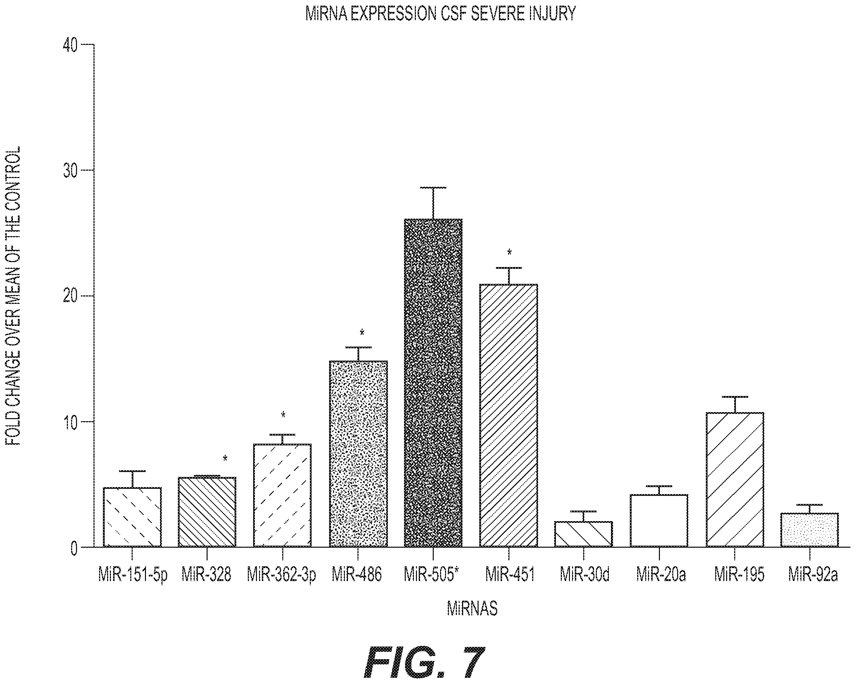

Figure 7 depicts additional miRNA specific validation assays in CSF samples of sTBI.

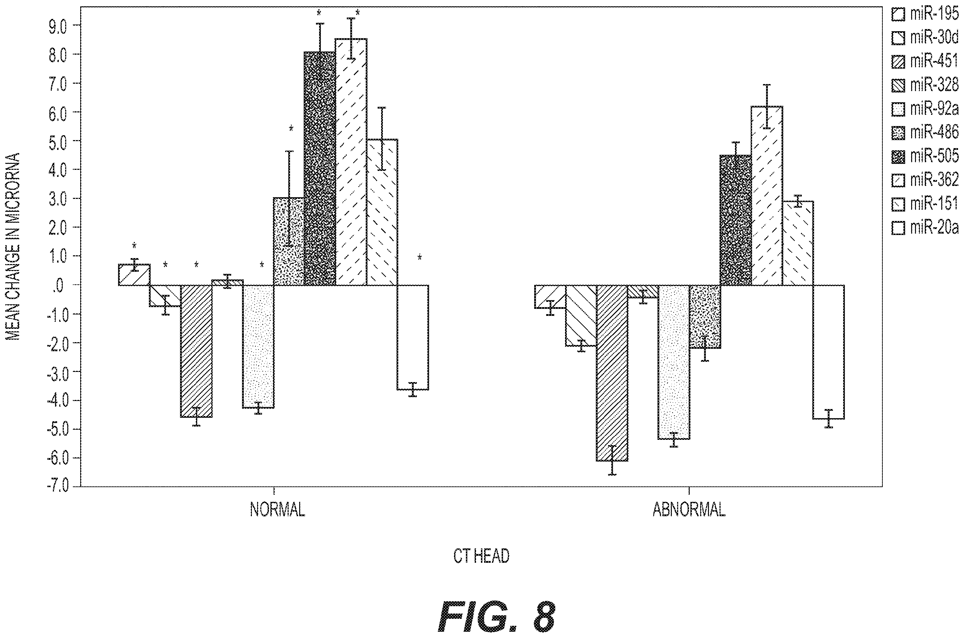

Figure 8 depicts levels of MicroRNA Biomarkers in those with head Ct lesions versus no head Ct lesions showing comparison of levels of miRNA in two groups of human subjects. Group 1 is comprised of subjects (TBI and controls) without any lesions on head CT (n = 19). Group 2 is TBI subjects with lesions on head CT (n = 12). The assumption was made that all controls (normal and trauma) had negative CT scans. There were significant differences between the two groups for all but two of the selected miRNA (see asterisks): miR-195 (p < 0.001); miR-30d (p < 0.001); miR-451 (p < 0.011); miR-328 (p = 0.101); miR-92a (p < 0.001); miR-486 (p = 0.006); miR-505 (p = 0.008); and miR-362 (p = 0.035); miR-151 (p = 0.065); and miR-20a (p = 0.012).

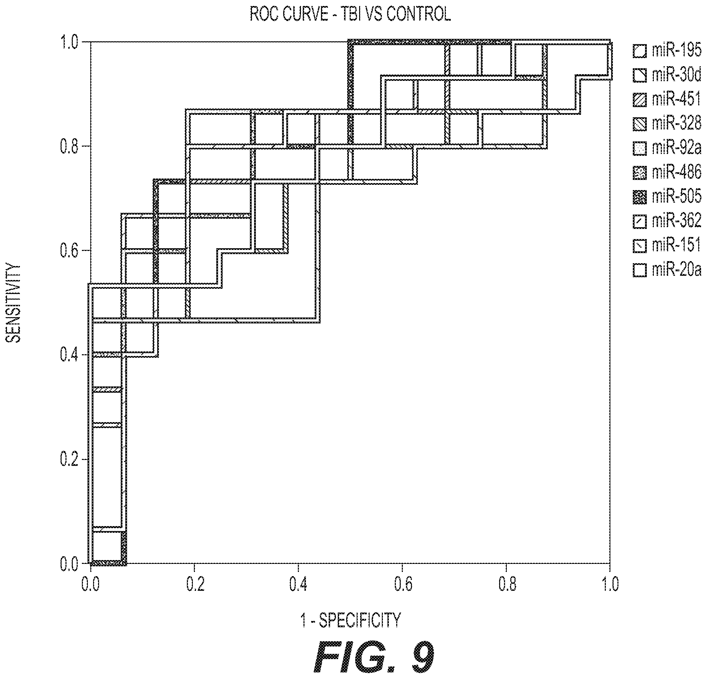

Figure 9 depicts that the diagnostic accuracy was assessed using the ROC Curve to determine the area under the curve for distinguishing TBI from controls. The AUC's were: miR-195 (0.81), miR-30d (0.75), miR-451 (0.82), miR-328 (0.73), miR-92a (0.86), miR-486 (0.81), miR-505 (0.82), miR-362 (0.79), miR-151 (0.66), miR-20a (0.78).

### Detailed Description of the Invention

The present disclosure relates to microRNA (miRNA) biomarkers from subjects with mild and severe traumatic brain injury (TBI), and their use thereof. MiRNAs are small RNA molecules (e.g. 22 nucleotides long) and are often, but need not be, post-transcriptional regulators that bind to complementary sequences on target messenger RNA transcripts (mRNAs), usually resulting in translational repression and gene silencing. MiRNAs may serve as good biomarkers because they are highly stable in serum due to their ability to withstand repeated freeze thaw, enzymatic degradation, and extreme pH conditions. As used herein, the term "microRNA" (miRNA) includes human miRNAs, mature single stranded miRNAs, precursor miRNAs (pre-miR), and variants thereof, which may be naturally occurring. In some instances, the term "miRNA" also includes primary miRNA transcripts and duplex miRNAs. Unless otherwise noted, when used herein, the name of a specific miRNA refers to the mature miRNA. For example, miR-194 refers to a mature miRNA sequence derived from pre-miR-194. The sequences for particular miRNAs, including human mature and precursor sequences, are reported, for example, in miRBase: Sequences Database on the web at: mirbase.org (version 20 released June 2013); Griffiths-Jones et al., Nucleic Acids Research, 2008, 36, Database Issue, D154-D158; Griffiths-Jones et al., Nucleic Acids Research, 2006, 34, Database Issue, D140-D144; Griffiths-Jones, Nucleic Acids Research, 2004, 32, Database Issue, D109-D111. For certain miRNAs, a single precursor contains more than one mature miRNA sequence. In other instances, multiple precursor miRNAs contain the same mature sequence. In some instances, mature miRNAs have been re-named based on new scientific consensus. The skilled artisan will appreciate that scientific consensus regarding the precise nucleic acid sequence for a given miRNAs, in particular for mature forms of the miRNAs, may change with time.

Disclosed herein are methods of diagnosing traumatic brain injury (TBI) in a subject. In some embodiments, the methods comprise (a) determining a level(s) of one or more miRNAs in a biological sample taken from the subject, and (b) comparing the determined level(s) of the one or more miRNAs against a level(s) of the same one or more miRNAs from a control subject determined not to be suffering from TBI. An increase in the level(s) of the one or more miRNAs compared to level(s) of the one or more miRNAs from the control subject determined not to be suffering from TBI may be indicative that the subject may be suffering from TBI.

Further disclosed herein are methods of monitoring the progression of traumatic brain injury (TBI) in a subject. In some embodiments, the method comprises (a) analyzing at least two biological samples from the subject taken at different time points to determine a level(s) of one or more specific miRNAs, and (b) comparing the level(s) of the one or more specific miRNAs over time to determine if the subject's level(s) of the one or more specific miRNAs is changing over time. An increase in the level(s) of the one or more specific miRNAs over time may be indicative that the subject's risk of suffering from TBI is increasing over time. In some embodiments, the level(s) of the one or more specific miRNAs may be normalized by the level(s) of one or more miRNA found to be consistent under various conditions. In some embodiments, the "one or more" miRNAs refer to one, two, three, four, five, six, seven, eight, nine, ten or more of miRNAs.

The term "diagnosing" includes making diagnostic or prognostic determinations or predictions of disease. In some instances, "diagnosing" includes identifying whether a subject has a disease such as TBI. Additionally, "diagnosing" includes distinguishing patients with mTBI from patients having sTBI. In other circumstances, "diagnosing" includes determining the stage or aggressiveness of a disease state, or determining an appropriate treatment method for TBI.

In some aspects, the methods of the present disclosure use miRNAs as markers for TBI. In some embodiments, miRNAs that are present at elevated levels in a biological sample (e.g. serum or plasma) from a subject with TBI are used as markers. In other embodiments, miRNAs that have reduced levels are used as markers. In some embodiments, more than one miRNA from the biological sample may be used as markers. When more than one miRNA biomarker is used, the miRNAs may all have elevated levels, all have reduced levels, or a mixture of miRNAs with elevated and reduced levels may be used.

The term "an increase in the level(s) of the one or more miRNAs" refers to an increase in the amount of a miRNA in a biological sample from a subject compared to the amount of the miRNA in the biological sample from a cohort or cohorts that do not have the TBI that the subject is being tested for. For instance, increased levels of miRNA in the biological sample indicate presence or prognosis for the TBI. In additional embodiments, certain miRNAs may be present in reduced levels in subjects with TBI. In some embodiments, the level of the miRNAs marker will be compared to a control to determine whether the level is decreased or increased. The control may be, for example, miRNAs in a biological sample from a subject known to be free of TBI. In other embodiments, the control may be miRNAs from a non-serum sample like a tissue sample or a known amount of a synthetic RNA. In additional embodiments, the control may be miRNAs in a biological sample from the same subject at a different time.

In one aspect, said miRNA is selected from the group consisting of hsa-miR-328, hsa-miR-362-3p, hsa-miR-486, hsa-miR-151-5p, hsa-miR-942, hsa-miR-194, hsa-miR-361, hsa-miR-625*, hsa-miR-1255B, hsa-miR-381, hsa-miR-425*, hsa-miR-638, hsa-miR-93, hsa-miR-1291, hsa-miR-19a, hsa-miR-601, hsa-miR-660, hsa-miR-9*, hsa-miR-130b, hsa-miR-339-3p, hsa-miR-34a, hsa-miR-455, hsa-miR-579, hsa-miR-624, mmu-miR-491, hsa-miR-195, hsa-miR-30d, hsa-miR-20a, hsa-miR-505*, mmu-miR-451, hsa-miR-199a-3p, hsa-miR-27a, hsa-miR-27b, hsa-miR-296, hsa-miR-92a and hsa-miR-29c. These miRNAs have elevated levels in serum from patients with TBI. Exemplary miRNAs are reported in Bhomia et al., Scientific Reports, 2016, 6, Article number: 28148. These miRNAs may be useful for diagnosing TBI, including distinguishing mild and sTBI. In some embodiments, said miRNAs exclude one, two, three, four, five, six, seven, eight or more, or all of hsa-miR-425*, hsa-miR-942, hsa-miR-361, hsa-miR-93, hsa-miR-34a, hsa-miR-455, hsa-miR-624, mmu-miR-491, and hsa-miR-27a.

In addition, these miRNA may be used to predict the aggressiveness or outcome of TBI. In another aspect, said one or more miRNAs is selected from the group consisting of hsa-miR-328, hsa-miR-151-5p, hsa-miR-362-3p, hsa-miR-486, hsa-miR-942, hsa-miR-194, hsa-miR-361, hsa-miR-625*, hsa-miR-1255B, hsa-miR-381, hsa-miR-425*, has-miR-638, hsa-miR-93, hsa-miR-195, hsa-miR-30d, hsa-miR-20a, hsa-miR-505*, mmu-miR-451, hsa-miR-199-3p, hsa-miR-27a, hsa-miR-92a and hsa-miR-27b. These miRNAs may be used to diagnose mTBI. In another aspect, said one or more miRNAs is selected from the group consisting of hsa-miR-328, hsa-miR-151-5p, hsa-miR-362-3p, hsa-miR-486, hsa-miR-942, hsa-miR-1291, hsa-miR-19a, hsa-miR-601, hsa-miR-660, hsa-miR-9*, miR-130b, hsa-miR-339-3p, hsa-miR-34a, hsa-miR-455, hsa-miR-579, hsa-miR-624, mmu-miR-491, hsa-miR-195, hsa-miR-30d, hsa-miR-20a, hsa-miR-505*, mmu-miR-451, hsa-miR-27a, hsa-miR-296, hsa-miR-92a and hsa-miR-29c. These miRNAs may be used to diagnose sTBI. In another aspect, said one or more miRNAs is selected from the group consisting of hsa-miR-328, hsa-miR-362-3p, hsa-miR-486, hsa-miR-151-5p, hsa-miR-942, hsa-miR-195, hsa-miR-30d, hsa-miR-20a, hsa-miR-505*, mmu-miR-451, hsa-miR-92a and hsa-miR-27a. These miRNAs may be used to diagnose either mTBI or sTBI. In some embodiments, said miRNAs exclude one, two, three, four, five, six, seven, eight or more, or all of hsa-miR-425*, hsa-miR-942, hsa-miR-361, hsa-miR-93, hsa-miR-34a, hsa-miR-455, hsa-miR-624, mmu-miR-491, and hsa-miR-27a.

In some embodiments, said one or more miRNAs is selected from the group consisting of hsa-miR-328, hsa-miR-151-5p, hsa-miR-362-3p, hsa-miR-486, hsa-miR-194, hsa-miR-625*, hsa-miR-1255B, hsa-miR-381, has-miR-638, hsa-miR-195, hsa-miR-30d, hsa-miR-20a, hsa-miR-505*, mmu-miR-451, hsa-miR-199-3p, hsa-miR-27a, hsa-miR-92a and hsa-miR-27b. These miRNAs may be used to diagnose mTBI. In another aspect, said one or more miRNAs is selected from the group consisting of hsa-miR-328, hsa-miR-151-5p, hsa-miR-362-3p, hsa-miR-486, hsa-miR-1291, hsa-miR-19a, hsa-miR-601, hsa-miR-660, hsa-miR-9*, miR-130b, hsa-miR-339-3p, hsa-miR-579, hsa-miR-195, hsa-miR-30d, hsa-miR-20a, hsa-miR-505*, mmu-miR-451, hsa-miR-296, hsa-miR-92a and hsa-miR-29c. These miRNAs may be used to diagnose sTBI. In another aspect, said one or more miRNAs is selected from the group consisting of hsa-miR-328, hsa-miR-362-3p, hsa-miR-486, hsa-miR-151-5p, hsa-miR-195, hsa-miR-30d, hsa-miR-20a, hsa-miR-505*, mmu-miR-451, and hsa-miR-92a. These miRNAs may be used to diagnose TBI, or either mTBI or sTBI.

In another aspect, the miRNAs comprise at least one, two or three miRNAs of miR-328, miR-362-3p and miR-486. For example, the methods may comprise assessing only miR-328, miR-362-3p and miR-486. In another embodiment, the methods comprise at least hsa-miR-328, hsa-miR-362-3p and hsa-miR-486, plus one or more of miR-151-5p, hsa-miR-942, hsa-miR-194, hsa-miR-361, hsa-miR-625*, hsa-miR-1255B, hsa-miR-381, hsa-miR-425*, hsa-miR-638, hsa-miR-93, hsa-hsa-miR-1291, hsa-miR-19a, hsa-miR-601, hsa-miR-660, hsa-miR-9*, hsa-miR-130b, hsa-miR-339-3p, hsa-miR-34a, hsa-miR-455, hsa-miR-579, hsa-miR-624, mmu-miR-491, hsa-miR-195, hsa-miR-30d, hsa-miR-20a, hsa-miR-505*, mmu-miR-451, hsa-miR-199a-3p, hsa-miR-27a, hsa-miR-27b, hsa-miR-296, hsa-miR-92a and hsa-miR-29c. In some embodiments, said miRNAs exclude one, two, three, four, five, six, seven, eight or more, or all of hsa-miR-425*, hsa-miR-942, hsa-miR-361, hsa-miR-93, hsa-miR-34a, hsa-miR-455, hsa-miR-624, mmu-miR-491, and hsa-miR-27a.

In another aspect, TBI may be classified as mTBI or sTBI. In some embodiments, the TBI is a closed head injury (CHI) or a blast-induced traumatic brain injury (bTBI).

In one aspect, injury severity may be based on duration of loss of consciousness and/or coma rating scale or score, post-traumatic amnesia (PTA), and/or brain imaging results. In some cases, mTBI may be characterized by brief loss of consciousness (e.g. a few seconds or minutes), PTA for less than 1 hour of the TBI, and normal brain imaging results. In additional embodiments, a case of mild traumatic brain injury may be an occurrence of injury to the head resulting from blunt trauma or acceleration or deceleration forces with one or more of the following conditions attributable to the head injury during the surveillance period: (i) any period of observed or self-reported transient confusion, disorientation, or impaired consciousness; (ii) any period of observed or self-reported dysfunction of memory (amnesia) around the time of injury; (iii) Observed signs of other neurological or neuropsychological dysfunction, such as seizures acutely following head injury, irritability, lethargy, or vomiting following head injury among infants and very young children, and among older children and adults, headache, dizziness, irritability, fatigue, or poor concentration, when identified soon after injury; and/or (iv) any period of observed or self-reported loss of consciousness lasting 30 minutes or less. In other cases, sTBI may be characterized by loss of consciousness or coma for more than 24 hours, PTA for more than 24 hours of the TBI, and/or abnormal brain imaging results.

In another aspect, the subject is human or animal. In another aspect, the biological samples described herein include, but is not limited to, homogenized tissues such as but not limited to brain tissue, spinal cord tissue, and tissue from specific regions of the central nervous system, blood, plasma, serum, urine, sputum, cerebrospinal fluid, milk, and ductal fluid samples. In some embodiments, the biological sample is a serum and/or plasma sample. Serum is typically the fluid, non-cellular portion of coagulated blood. Plasma is also a non-cellular blood sample, but unlike serum, plasma contains clotting factors. In some embodiments, serum or plasma samples may be obtained from a human subject previously screened for TBI using other diagnostic methods. Additional embodiments include measuring miRNA in samples from subjects previously or currently undergoing treatment for TBI. The volume of plasma or serum obtained and used in the methods described herein may be varied depending upon clinical intent.

One of skill in the art may recognize that many methods exist for obtaining and preparing serum samples. Generally, blood is drawn into a collection tube using standard methods and allowed to clot. The serum is then separated from the cellular portion of the coagulated blood. In some embodiments clotting activators such as silica particles are added to the blood collection tube. In other methods, the blood is not treated to facilitate clotting. Blood collection tubes are commercially available from many sources and in a variety of formats (e.g., Becton Dickenson Vacutainer® tubes-SST™, glass serum tubes, or plastic serum tubes).

In some embodiments, the blood is collected by venipuncture and processed within three hours after drawing to minimize hemolysis and minimize the release of miRNAs from intact cells in the blood. In some methods, blood is kept on ice until use. The blood may be fractionated by centrifugation to remove cellular components. In some embodiments, centrifugation to prepare serum can be at a speed of at least 500, 1000, 2000, 3000, 4000, or 5000×G. In certain embodiments, the blood can be incubated for at least 10, 20, 30, 40, 50, 60, 90, 120, or 150 minutes to allow clotting. In other embodiments, the blood is incubated for at most 3 hours. When using plasma, the blood is not permitted to coagulate prior to separation of the cellular and acellular components. Serum or plasma may be frozen after separation from the cellular portion of blood until further assayed.

Before performing the methods according to the present disclosure RNA may be extracted from biological samples such as but not limited to serum or plasma and purified using methods known in the art. Many methods are known for isolating total RNA, or to specifically extract small RNAs, including miRNAs. The RNA may be extracted using commercially-available kits (e.g., Perfect RNA Total RNA Isolation Kit, Five Prime-Three Prime, Inc.; mirVana™ kits, Ambion, Inc.). Alternatively, RNA extraction methods previously published for the extraction of mammalian intracellular RNA or viral RNA may be adapted, either as published or with modification, for extraction of RNA from plasma and serum. RNA may be extracted from plasma or serum using silica particles, glass beads, or diatoms, as in the method or adaptations described in U.S. Publication No. 2008/0057502.

In another aspect, the biological sample may be collected from a subject more than 1, 2, 3, 4, 5, 6, 7, 8, 9, 10, 11, 12, 13, or 14 days after a suspected traumatic episode. In another aspect, the biological sample may be collected from a subject less than 1, 2, 3, 4, 5, 6, 7, 8, 9, 10, 11, 12, 13, 14, or 15 days after a suspected traumatic episode.

In another aspect, the level(s) of one or more specific miRNAs are determined by a real time PCR. In some embodiments, the methods of the present inventions comprise amplifying the miRNAs.

Many methods of measuring the levels or amounts of miRNAs are contemplated. Any reliable, sensitive, and specific method may be used. In some embodiments, the miRNAs are amplified prior to measurement. In other embodiments, the level of miRNAs is measured during the amplification process. In still other methods, the miRNAs is not amplified prior to measurement.

Many methods exist for amplifying miRNA nucleic acid sequences such as mature miRNAs, primary miRNAs and precursor miRNAs. Suitable nucleic acid polymerization and amplification techniques include reverse transcription (RT), polymerase chain reaction (PCR), real-time PCR (quantitative PCR (q-PCR)), nucleic acid sequence-base amplification (NASBA), ligase chain reaction, multiplex ligatable probe amplification, invader technology (Third Wave), rolling circle amplification, in vitro transcription (IVT), strand displacement amplification, transcription-mediated amplification (TMA), RNA (Eberwine) amplification, and other methods that are known to persons skilled in the art. In certain embodiments, more than one amplification method is used, such as reverse transcription followed by real time quantitative PCR (qRT-PCR) (Chen et al., Nucleic Acids Research, 33(20):e179 (2005)).

A typical PCR reaction includes multiple amplification steps, or cycles that selectively amplify target nucleic acid species: a denaturing step in which a target nucleic acid is denatured; an annealing step in which a set of PCR primers (forward and reverse primers) anneal to complementary DNA strands; and an elongation step in which a thermostable DNA polymerase elongates the primers. By repeating these steps multiple times, a DNA fragment is amplified to produce an amplicon, corresponding to the target DNA sequence. Typical PCR reactions include 20 or more cycles of denaturation, annealing, and elongation. In many cases, the annealing and elongation steps can be performed concurrently, in which case the cycle contains only two steps. Since mature miRNAs are single-stranded, a reverse transcription reaction (which produces a complementary cDNA sequence) may be performed prior to PCR reactions. Reverse transcription reactions include the use of, e.g., a RNA-based DNA polymerase (reverse transcriptase) and a primer.

In PCR and q-PCR methods, for example, a set of primers is used for each target sequence. In certain embodiments, the lengths of the primers depends on many factors, including, but not limited to, the desired hybridization temperature between the primers, the target nucleic acid sequence, and the complexity of the different target nucleic acid sequences to be amplified. In certain embodiments, a primer is about 15 to about 35 nucleotides in length. In other embodiments, a primer is equal to or fewer than 15, 20, 25, 30, or 35 nucleotides in length. In additional embodiments, a primer is at least 35 nucleotides in length.

In a further aspect, a forward primer can comprise at least one sequence that anneals to a miRNA biomarker and alternatively can comprise an additional 5' non-complementary region. In another aspect, a reverse primer can be designed to anneal to the complement of a reverse transcribed miRNAs. The reverse primer may be independent of the miRNA biomarker sequence, and multiple miRNA biomarkers may be amplified using the same reverse primer. Alternatively, a reverse primer may be specific for a miRNA biomarker.

In some embodiments, two or more miRNAs are amplified in a single reaction volume. One aspect includes multiplex q-PCR, such as Real Time quantitative PCR (qRT-PCR), which enables simultaneous amplification and quantification of at least two miRNAs of interest in one reaction volume by using more than one pair of primers and/or more than one probe. The primer pairs comprise at least one amplification primer that uniquely binds each miRNA, and the probes are labeled such that they are distinguishable from one another, thus allowing simultaneous quantification of multiple miRNAs. Multiplex qRT-PCR has research and diagnostic uses, including but not limited to detection of miRNAs for diagnostic, prognostic, and therapeutic applications.

The qRT-PCR reaction may further be combined with the reverse transcription reaction by including both a reverse transcriptase and a DNA-based thermostable DNA polymerase. When two polymerases are used, a "hot start" approach may be used to maximize assay performance (U.S. Pat. Nos. 5,411,876 and 5,985,619). For example, the components for a reverse transcriptase reaction and a PCR reaction may be sequestered using one or more thermoactivation methods or chemical alteration to improve polymerization efficiency (U.S. Pat. Nos. 5,550,044, 5,413,924, and 6,403,341).

In certain embodiments, labels, dyes, or labeled probes and/or primers are used to detect amplified or unamplified miRNAs. The skilled artisan will recognize which detection methods are appropriate based on the sensitivity of the detection method and the abundance of the target. Depending on the sensitivity of the detection method and the abundance of the target, amplification may or may not be required prior to detection. One skilled in the art will recognize the detection methods where miRNA amplification is preferred.

A probe or primer may include Watson-Crick bases or modified bases. Modified bases include, but are not limited to, the AEGIS bases (from Eragen Biosciences), which have been described, e.g., in U.S. Pat. Nos. 5,432,272, 5,965,364, and 6,001,983. In certain aspects, bases are joined by a natural phosphodiester bond or a different chemical linkage. Different chemical linkages include, but are not limited to, a peptide bond or a Locked Nucleic Acid (LNA) linkage, which is described, e.g., in U.S. Pat. No. 7,060,809.

In a further aspect, oligonucleotide probes or primers present in an amplification reaction are suitable for monitoring the amount of amplification product produced as a function of time. In certain aspects, probes having different single stranded versus double stranded character are used to detect the nucleic acid. Probes include, but are not limited to, the 5'-exonuclease assay (e.g., TaqMan™) probes (see U.S. Pat. No. 5,538,848), stem-loop molecular beacons (see, e.g., U.S. Pat. Nos. 6,103,476 and 5,925,517), stemless or linear beacons (see, e.g., WO 9921881, U.S. Pat. Nos. 6,485,901 and 6,649,349), peptide nucleic acid (PNA) Molecular Beacons (see, e.g., U.S. Pat. Nos. 6,355,421 and 6,593,091), linear PNA beacons (see, e.g. U.S. Pat. No. 6,329,144), non-FRET probes (see, e.g., U.S. Pat. No. 6,150,097), Sunrise™/AmplifluorB™probes (see, e.g., U.S. Pat. No. 6,548,250), stem-loop and duplex Scorpion™ probes (see, e.g., U.S. Pat. No. 6,589,743), bulge loop probes (see, e.g., U.S. Pat. No. 6,590,091), pseudo knot probes (see, e.g., U.S. Pat. No. 6,548,250), cyclicons (see, e.g., U.S. Pat. No. 6,383,752), MGB Eclipse™ probe (Epoch Biosciences), hairpin probes (see, e.g., U.S. Pat. No. 6,596,490), PNA light-up probes, antiprimer quench probes (Li et al., Clin. Chem. 53:624-633 (2006)), self-assembled nanoparticle probes, and ferrocene-modified probes described, for example, in U.S. Pat. No. 6,485,901.

In certain embodiments, one or more of the primers in an amplification reaction can include a label. In yet further embodiments, different probes or primers comprise detectable labels that are distinguishable from one another. In some embodiments a nucleic acid, such as the probe or primer, may be labeled with two or more distinguishable labels.

In some aspects, a label is attached to one or more probes and has one or more of the following properties: (i) provides a detectable signal; (ii) interacts with a second label to modify the detectable signal provided by the second label, e.g., FRET (Fluorescent Resonance Energy Transfer); (iii) stabilizes hybridization, e.g., duplex formation; and (iv) provides a member of a binding complex or affinity set, e.g., affinity, antibody-antigen, ionic complexes, hapten-ligand (e.g., biotin-avidin). In still other aspects, use of labels can be accomplished using any one of a large number of known techniques employing known labels, linkages, linking groups, reagents, reaction conditions, and analysis and purification methods.

MiRNAs can be detected by direct or indirect methods. In a direct detection method, one or more miRNAs are detected by a detectable label that is linked to a nucleic acid molecule. In such methods, the miRNAs may be labeled prior to binding to the probe. Therefore, binding is detected by screening for the labeled miRNAs that is bound to the probe. The probe is optionally linked to a bead in the reaction volume.

In certain embodiments, nucleic acids are detected by direct binding with a labeled probe, and the probe is subsequently detected. In one embodiment of the present invention, the nucleic acids, such as amplified miRNAs, are detected using FIexMAP Microspheres (Luminex) conjugated with probes to capture the desired nucleic acids.

Some methods may involve detection with polynucleotide probes modified with fluorescent labels or branched DNA (bDNA) detection, for example.

In other embodiments, nucleic acids are detected by indirect detection methods. For example, a biotinylated probe may be combined with a streptavidin-conjugated dye to detect the bound nucleic acid. The streptavidin molecule binds a biotin label on amplified miRNAs, and the bound miRNA is detected by detecting the dye molecule attached to the streptavidin molecule. In one embodiment, the streptavidin-conjugated dye molecule comprises Phycolink® Streptavidin R-Phycoerythrin (PROzyme). Other conjugated dye molecules are known to persons skilled in the art.

Labels include, but are not limited to: light-emitting, light-scattering, and light-absorbing compounds which generate or quench a detectable fluorescent, chemiluminescent, or bioluminescent signal (see, e.g., Kricka, L., Nonisotopic DNA Probe Techniquies, Academic Press, San Diego (1992) and Garman A., Non-Radioactive Labeling, Academic Press (1997). Fluorescent reporter dyes useful as labels include, but are not limited to, fluoresceins (see, e.g., U.S. Pat. Nos. 5,188,934, 6,008,379, and 6,020,481), rhodamines (see, e.g., U.S. Pat. Nos. 5,366,860, 5,847,162, 5,936,087, 6,051,719, and 6,191,278), benzophenoxazines (see, e.g., U.S. Pat. No. 6,140,500), energy-transfer fluorescent dyes, comprising pairs of donors and acceptors (see, e.g., U.S. Pat. Nos. 5,863,727; 5,800,996; and 5,945,526), and cyanines (see, e.g., WO 9745539), lissamine, phycoerythrin, Cy2, Cy3, Cy3.5, Cy5, Cy5.5, Cy7, FluorX (Amersham), Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, Tetramethylrhodamine, and/or Texas Red, as well as any other fluorescent moiety capable of generating a detectable signal. Examples of fluorescein dyes include, but are not limited to, 6-carboxyfluorescein, 2',4',1,4,-tetrachlorofluorescein and 2',4',5',7',1,4-hexachlorofluorescein. In certain aspects, the fluorescent label is selected from SYBR-Green, 6-carboxyfluorescein ("FAM"), TET, ROX, VICTM, and JOE. For example, in certain embodiments, labels are different fluorophores capable of emitting light at different, spectrally-resolvable wavelengths (e.g., 4-differently colored fluorophores); certain such labeled probes are known in the art and described above, and in U.S. Pat. No. 6,140,054. A dual labeled fluorescent probe that includes a reporter fluorophore and a quencher fluorophore is used in some embodiments. It will be appreciated that pairs of fluorophores are chosen that have distinct emission spectra so that they can be easily distinguished.

In still a further aspect, labels are hybridization-stabilizing moieties which serve to enhance, stabilize, or influence hybridization of duplexes, e.g., intercalators and intercalating dyes (including, but not limited to, ethidium bromide and SYBR-Green), minor-groove binders, and cross-linking functional groups (see, e.g., Blackburn et al., eds. "DNA and RNA Structure" in Nucleic Acids in Chemistry and Biology (1996)).

In further aspects, methods relying on hybridization and/or ligation to quantify miRNAs may be used, including oligonucleotide ligation (OLA) methods and methods that allow a distinguishable probe that hybridizes to the target nucleic acid sequence to be separated from an unbound probe. As an example, HARP-like probes, as disclosed in U.S. Publication No. 2006/0078894 may be used to measure the amount of miRNAs. In such methods, after hybridization between a probe and the targeted nucleic acid, the probe is modified to distinguish the hybridized probe from the unhybridized probe. Thereafter, the probe may be amplified and/or detected. In general, a probe inactivation region comprises a subset of nucleotides within the target hybridization region of the probe. To reduce or prevent amplification or detection of a HARP probe that is not hybridized to its target nucleic acid, and thus allow detection of the target nucleic acid, a post-hybridization probe inactivation step is carried out using an agent which is able to distinguish between a HARP probe that is hybridized to its targeted nucleic acid sequence and the corresponding unhybridized HARP probe. The agent is able to inactivate or modify the unhybridized HARP probe such that it cannot be amplified.

In an additional embodiment of the method, a probe ligation reaction may be used to quantify miRNAs. In a Multiplex Ligation-dependent Probe Amplification (MLPA) technique (Schouten et al., Nucleic Acids Research 30:e57 (2002)), pairs of probes which hybridize immediately adjacent to each other on the target nucleic acid are ligated to each other only in the presence of the target nucleic acid. In some aspects, MLPA probes have flanking PCR primer binding sites. MLPA probes can only be amplified if they have been ligated, thus allowing for detection and quantification of miRNA biomarkers.

### Examples

The following examples illustrate various embodiments of the present disclosure and are not intended to limit the scope of the invention, which is limited by the appended claims.

### Experiment 1

Global normalization on the miRNA expression data of samples from subjects with mild TBI (mTBI), severe TBI (sTBI), and orthopedic injury to control samples was performed, and candidates for each group were identified. Human serum samples were collected from each of subjects with mTBI (n=8), sTBI (n=8), and orthopedic injury (n=7). The mTBI samples were collected within 24hr of injury and sTBI samples were collected within 48hr of injury. Control samples (n=8) were also collected from healthy control subjects.

RNA isolation was performed using miRNeasy Serum/Plasma Kit (Qiagen Inc). For RNA quality control, all total RNA samples were analyzed with the Agilent Small RNA kit (Agilent Technologies Inc, Santa Clara, CA, USA) to measure the small RNA/ miRNA concentration. Reverse transcription (RT) was performed with TaqMan miRNA RT Kit (Life Technologies, Carlsbad, CA, USA) and miRNA quantity was measured from the total RNA of bioanalyzer data and used as template RNA (3µl out of 16 µl total eluted RNA)) for RT reactions. Pre-amplification of the cDNA product after RT was done using 12.5 µl TaqMan PreAmp Master Mix, 2.50 µl Megaplex PreAmp primers human Pool A/B (v3.0), 5 µl of nuclease-free water and 5µl of RT product to make up a final volume of 25 µl of final reaction mixture.

Real time PCR was performed for a set of 792 human miRNAs for serum samples of mild (n=8), severe (n=8), orthopedic injury (n=7) and healthy controls (n=8). PCR was carried out with the TaqMan Low Density Human MicroRNA array cards (TLDA) and using default thermal-cycling conditions in AB7900 Real Time HT machine (Applied Biosystem). PCR amplification of the serum miRNAs detected more than 140 miRNAs in the control serum samples. For relative quantization of miRNAs in serum samples, a stable endogenous control is a major limitation. To analyze the real time PCR miRNA data, a global normalization algorithm was used which calculates a reference endogenous control based on the overall amplification of the miRNAs in the same plate. This method has been widely accepted as a way of normalization for multiplexing assays in serum samples.

The normalized delta Ct values were used to perform hierarchical clustering to understand pattern of expression between the experimental groups. Hierarchical clustering segregated the study under four differentially expressing groups which belonged to control, orthopedic injury and the TBI groups suggesting a clear difference in miRNA expression between these experimental groups (Figure 1). After the normalization, the fold change for the serum miRNAs in mTBI, sTBI and orthopedic injury groups was calculated using healthy control subjects as baseline. MiRNAs with more than 2 fold upregulation and adjusted p value ≤0.05 were selected for further analysis. From this analysis, it was found that in serum samples of mTBI and sTBI, 39 and 37 miRNAs were significantly upregulated respectively whereas 33 miRNAs were found to be modulated in orthopedic injury group as shown in Tables 1-3.

**Table 1: Total MiRNAs altered in serum samples of MTBI after normalizing with healthy controls. Data was normalized using global normalization and was compared with healthy controls. Data was adjusted for multiple comparisons using adjusted p value <0.05 calculated using Benjamin Hochberg algorithm.**

| **MTBI vs Control** | | | | | |
|---|---|---|---|---|---|
| S# | Detector | RQ_ mTBI-Control | adj.P.Val_ mTBI-Control | P.Value_ mTBI-Control | GeneSymbol |
| 1 | hsa-miR-381-000571 | 2255.75 | 0.01 | 0.01 | hsa-miR-381 |
| 2 | hsa-miR-185-002271 | 605.52 | 0.00 | 0.00 | hsa-miR-185 |
| 3 | hsa-miR-486-001278 | 523.46 | 0.01 | 0.00 | hsa-miR-486 |
| 4 | hsa-miR-532-001518 | 492.81 | 0.00 | 0.00 | hsa-miR-532 |
| 5 | hsa-miR-423-5p-002340 | 415.56 | 0.00 | 0.00 | hsa-miR-423 |
| 6 | hsa-miR-193a-5p-002281 | 221.14 | 0.00 | 0.00 | hsa-miR-193a |
| 7 | hsa-miR-133a-002246 | 75.25 | 0.02 | 0.01 | hsa-miR-133a |
| 8 | hsa-miR-638-001582 | 46.48 | 0.05 | 0.03 | hsa-miR-638 |
| 9 | hsa-miR-151-5P-002642 | 45.52 | 0.03 | 0.02 | hsa-miR-151 |
| 10 | hsa-miR-223#-002098 | 42.61 | 0.01 | 0.01 | hsa-miR-223 |
| 11 | hsa-miR-625#-002432 | 40.51 | 0.03 | 0.03 | hsa-miR-625 |
| 12 | hsa-miR-505#-002087 | 33.39 | 0.04 | 0.03 | hsa-miR-505 |
| 13 | hsa-miR-194-000493 | 31.43 | 0.04 | 0.03 | hsa-miR-194 |
| 14 | hsa-miR-576-3p-002351 | 25.40 | 0.02 | 0.01 | hsa-miR-576 |
| 15 | hsa-miR-1255B-002801 | 19.19 | 0.01 | 0.00 | hsa-miR-1255B |
| 16 | hsa-miR-362-3p-002117 | 14.54 | 0.01 | 0.01 | hsa-miR-362 |
| 17 | hsa-miR-409-3p-002332 | 12.83 | 0.02 | 0.01 | hsa-miR-409 |
| 18 | mmu-miR-451-001141 | 8.37 | 0.00 | 0.00 | mmu-miR-451 |
| 19 | hsa-miR-16-000391 | 7.44 | 0.00 | 0.00 | hsa-miR-16 |
| 20 | hsa-miR-365-001020 | 6.76 | 0.01 | 0.01 | hsa-miR-365 |
| 21 | hsa-miR-25-000403 | 6.71 | 0.00 | 0.00 | hsa-miR-25 |
| 22 | hsa-miR-151-3p-002254 | 6.61 | 0.02 | 0.01 | hsa-miR-151 |
| 23 | hsa-miR-376c-002122 | 5.21 | 0.00 | 0.00 | hsa-miR-376c |
| 24 | hsa-miR-21-000397 | 4.95 | 0.00 | 0.00 | hsa-miR-21 |
| 25 | hsa-miR-146a-000468 | 4.25 | 0.00 | 0.00 | hsa-miR-146a |
| 26 | hsa-miR-20a-000580 | 4.19 | 0.00 | 0.00 | hsa-miR-20a |
| 27 | hsa-miR-484-001821 | 3.89 | 0.00 | 0.00 | hsa-miR-484 |
| 28 | hsa-miR-92a-000431 | 3.77 | 0.00 | 0.00 | hsa-miR-92a |
| 29 | hsa-miR-152-000475 | 3.64 | 0.00 | 0.00 | hsa-miR-152 |
| 30 | hsa-miR-590-5p-001984 | 3.27 | 0.04 | 0.03 | hsa-miR-590 |
| 31 | hsa-miR-199a-3p-002304 | 3.02 | 0.00 | 0.00 | hsa-miR-199a |
| 32 | hsa-miR-30d-000420 | 2.92 | 0.00 | 0.00 | hsa-miR-30d |
| 33 | hsa-miR-223-002295 | 2.65 | 0.02 | 0.02 | hsa-miR-223 |
| 34 | hsa-miR-186-002285 | 2.57 | 0.00 | 0.00 | hsa-miR-186 |
| 35 | hsa-miR-328-000543 | 2.56 | 0.00 | 0.00 | hsa-miR-328 |
| 36 | hsa-miR-27b-000409 | 2.51 | 0.00 | 0.00 | hsa-miR-27b |
| 37 | hsa-miR-195-000494 | 2.46 | 0.01 | 0.01 | hsa-miR-195 |
| 38 | hsa-miR-27a-000408 | 2.06 | 0.00 | 0.00 | hsa-miR-27a |
| 39 | hsa-miR-19b-000396 | 2.06 | 0.04 | 0.03 | hsa-miR-19b |

**Table 2: Total MiRNAs altered in serum samples of STBI after normalizing with healthy controls. Data was normalized using global normalization and was compared with healthy controls. Data was adjusted for multiple comparisons using adjusted p value <0.05 calculated using Benjamin Hochberg algorithm.**

| **sTBI vs Control** | | | | | |
|---|---|---|---|---|---|
| S# | Detector | RQ_sTBI-Control | adj.P.Val_sTBI-Control | P.Value_sTBI-Control | GeneSymbol |
| 1 | hsa-miR-193a-5p-002281 | 476.64 | 0.00 | 0.00 | hsa-miR-193a |
| 2 | hsa-miR-486-001278 | 281.67 | 0.01 | 0.01 | hsa-miR-486 |
| 3 | hsa-miR-423-5p-002340 | 207.23 | 0.01 | 0.00 | hsa-miR-423 |
| 4 | hsa-miR-532-001518 | 202.24 | 0.01 | 0.01 | hsa-miR-532 |
| 5 | hsa-miR-185-002271 | 92.99 | 0.04 | 0.03 | hsa-miR-185 |
| 6 | hsa-miR-133a-002246 | 82.04 | 0.01 | 0.01 | hsa-miR-133a |
| 7 | hsa-miR-576-3p-002351 | 74.38 | 0.00 | 0.00 | hsa-miR-576 |
| 8 | hsa-miR-130b-000456 | 59.04 | 0.04 | 0.03 | hsa-miR-130b |
| 9 | hsa-miR-296-000527 | 43.17 | 0.02 | 0.01 | hsa-miR-296 |
| 10 | hsa-miR-505#-002087 | 36.62 | 0.01 | 0.00 | hsa-miR-505 |
| 11 | hsa-miR-223#-002098 | 34.41 | 0.02 | 0.01 | hsa-miR-223 |
| 12 | hsa-miR-151-5P-002642 | 29.71 | 0.05 | 0.03 | hsa-miR-151 |
| 13 | hsa-miR-579-002398 | 18.64 | 0.01 | 0.01 | hsa-miR-579 |
| 14 | hsa-miR-339-3p-002184 | 14.00 | 0.03 | 0.02 | hsa-miR-339 |
| ***15** | hsa-miR-362-3p-002117 | 13.74 | 0.05 | 0.04 | hsa-miR-362 |
| 16 | hsa-miR-365-001020 | 12.41 | 0.00 | 0.00 | hsa-miR-365 |
| 17 | hsa-miR-29a-002112 | 6.90 | 0.00 | 0.00 | hsa-miR-29a |
| 18 | hsa-miR-19a-000395 | 5.53 | 0.02 | 0.01 | hsa-miR-19a |
| 19 | hsa-miR-9#-002231 | 4.74 | 0.00 | 0.00 | hsa-miR-9 |
| 20 | hsa-miR-30d-000420 | 4.56 | 0.00 | 0.00 | hsa-miR-30d |
| 21 | hsa-miR-25-000403 | 4.22 | 0.00 | 0.00 | hsa-miR-25 |
| 22 | hsa-miR-601-001558 | 4.12 | 0.03 | 0.02 | hsa-miR-601 |
| 23 | hsa-miR-16-000391 | 4.01 | 0.00 | 0.00 | hsa-miR-16 |
| 24 | hsa-miR-1291-002838 | 3.72 | 0.02 | 0.01 | hsa-miR-1291 |
| 25 | hsa-miR-21-000397 | 3.69 | 0.00 | 0.00 | hsa-miR-21 |
| 26 | hsa-miR-195-000494 | 3.52 | 0.00 | 0.00 | hsa-miR-195 |
| 27 | hsa-miR-146a-000468 | 3.12 | 0.01 | 0.00 | hsa-miR-146a |
| 28 | hsa-miR-660-001515 | 2.84 | 0.01 | 0.01 | hsa-miR-660 |
| 29 | hsa-miR-29c-000587 | 2.80 | 0.01 | 0.00 | hsa-miR-29c |
| 30 | hsa-miR-19b-000396 | 2.63 | 0.01 | 0.00 | hsa-miR-19b |
| 31 | mmu-miR-451-001141 | 2.57 | 0.05 | 0.03 | mmu-miR-451 |
| 32 | hsa-miR-92a-000431 | 2.57 | 0.00 | 0.00 | hsa-miR-92a |
| 33 | hsa-miR-186-002285 | 2.56 | 0.02 | 0.02 | hsa-miR-186 |
| 34 | hsa-miR-484-001821 | 2.49 | 0.00 | 0.00 | hsa-miR-484 |
| 35 | hsa-miR-20a-000580 | 2.31 | 0.01 | 0.01 | hsa-miR-20a |
| 36 | hsa-miR-24-000402 | 2.25 | 0.00 | 0.00 | hsa-miR-24 |
| 37 | hsa-miR-328-000543 | 2.02 | 0.02 | 0.01 | hsa-miR-328 |

**Table 3: Total MiRNAs altered in serum samples of Orthopedic Injury group after normalizing with healthy controls. Data was normalized using global normalization and was compared with healthy controls. Data was adjusted for multiple comparisons using adjusted p value <0.05 calculated using Benjamin Hochberg algorithm.**

| **Ortho vs Control** | | | | | |
|---|---|---|---|---|---|
| S# | Detector | RQ_Ortho-Control | adj.P.Val_Ortho-Control | P.Value_Ortho-Control | GeneSymbol |
| 1 | hsa-miR-520c-3p-002400 | 23063.46 | 0.02 | 0.00 | hsa-miR-520c |
| 2 | hsa-miR-155-002623 | 941.78 | 0.01 | 0.00 | hsa-miR-155 |
| 3 | hsa-miR-185-002271 | 467.20 | 0.03 | 0.00 | hsa-miR-185 |
| 4 | hsa-miR-766-001986 | 425.82 | 0.01 | 0.00 | hsa-miR-766 |
| 5 | hsa-miR-532-001518 | 366.99 | 0.01 | 0.00 | hsa-miR-532 |
| 6 | hsa-miR-193a-5p-002281 | 322.15 | 0.01 | 0.00 | hsa-miR-193a |
| 7 | hsa-miR-423-5p-002340 | 216.43 | 0.03 | 0.00 | hsa-miR-16 |
| 8 | hsa-miR-132-000457 | 197.50 | 0.03 | 0.01 | hsa-miR-132 |
| 9 | hsa-miR-133a-002246 | 49.67 | 0.03 | 0.02 | hsa-miR-133a |
| 10 | hsa-miR-223#-002098 | 42.32 | 0.03 | 0.01 | hsa-miR-223 |
| 11 | hsa-miR-642-001592 | 27.33 | 0.03 | 0.02 | hsa-miR-642 |
| 12 | hsa-miR-576-3p-002351 | 22.18 | 0.04 | 0.02 | hsa-miR-576 |
| 13 | hsa-miR-409-3p-002332 | 16.73 | 0.04 | 0.03 | hsa-miR-409 |
| 14 | hsa-miR-375-000564 | 16.69 | 0.03 | 0.02 | hsa-miR-375 |
| 15 | hsa-miR-146a-000468 | 12.84 | 0.00 | 0.00 | hsa-miR-146a |
| 16 | hsa-miR-29a-002112 | 10.45 | 0.03 | 0.01 | hsa-miR-29a |
| 17 | hsa-miR-186-002285 | 9.91 | 0.03 | 0.01 | hsa-miR-186 |
| 18 | hsa-miR-376c-002122 | 8.41 | 0.02 | 0.00 | hsa-miR-376c |
| 19 | hsa-miR-197-000497 | 6.62 | 0.00 | 0.00 | hsa-miR-197 |
| 20 | hsa-miR-365-001020 | 6.16 | 0.03 | 0.00 | hsa-miR-365 |
| 21 | hsa-miR-222-002276 | 5.57 | 0.01 | 0.00 | hsa-miR-222 |
| 22 | mmu-miR-374-5p-001319 | 5.29 | 0.03 | 0.00 | mmu-miR-374 |
| 23 | hsa-miR-21-000397 | 4.55 | 0.03 | 0.00 | hsa-miR-21 |
| 24 | hsa-miR-16-000391 | 4.43 | 0.03 | 0.00 | hsa-miR-409 |
| 25 | hsa-miR-192-000491 | 4.30 | 0.03 | 0.01 | hsa-miR-192 |
| 26 | hsa-miR-484-001821 | 4.23 | 0.01 | 0.00 | hsa-miR-484 |
| 27 | hsa-miR-25-000403 | 4.16 | 0.02 | 0.00 | hsa-miR-25 |
| 28 | hsa-miR-223-002295 | 4.05 | 0.03 | 0.01 | hsa-miR-223 |
| 29 | hsa-miR-151-3p-002254 | 3.56 | 0.03 | 0.01 | hsa-miR-151 |
| 30 | hsa-miR-590-5p-001984 | 3.50 | 0.05 | 0.03 | hsa-miR-590 |
| 31 | hsa-miR-24-000402 | 3.48 | 0.01 | 0.00 | hsa-miR-24 |
| 32 | hsa-miR-152-000475 | 2.97 | 0.03 | 0.01 | hsa-miR-152 |
| 33 | hsa-miR-19b-000396 | 2.60 | 0.03 | 0.01 | hsa-miR-19b |

The real time PCR results of the samples from the subjects with the mTBI, sTBI and orthopedic injury were normalized to the real time PCR result of the control sample. Our analysis showed that 82, 74 and 58 miRNAs were significantly modulated in serum samples from the subjects with the mTBI, sTBI and orthopedic injury, respectively. The levels of the miRNAs in the samples from the subjects with the mTBI and sTBI were compared to the level of the miRNAs in the sample from the subjects with the orthopedic injury. The results showed upregulation of 22 and 26 miRNAs in the samples from the subjects with mTBI and sTBI compared to the modulated level of the miRNAs in the sample from the subjects with the orthopedic injury. These 22 unique miRNAs for mTBI and 26 unique miRNAs for sTBI are listed in Tables 4 and 5 along with their normalized fold changes indicating their level of expression.

**Table 4: MiRNAs altered in serum samples of mTBI.**

| S# | Micro RNA | Fold Change | Mature Sequence | Mirbase ID |
|---|---|---|---|---|
| 1. | hsa-miR-381 | 2238.72 | UAUACAAGGGCAAGCUCUCUGU | MIMAT0000736 |
| 2. | hsa-miR-425* | 645.65 | AUCGGGAAUGUCGUGUCCGCCC | MIMAT0001343 |
| 3. | hsa-miR-486 | 523.46 | UCCUGUACUGAGCUGCCCCGAG | MIMAT0002177 |
| 4. | hsa-miR-942 | 424.19 | UCUUCUCUGUUUUGGCCAUGUG | MIMA T0004985 |
| 5. | hsa-miR-638 | 46.48 | AGGGAUCGCGGGCGGGUGGCGGCCU | MIMAT0003 308 |
| 6. | hsa-miR-151-5p | 45.52 | UCGAGGAGCUCACAGUCUAGU | MIMA T0004697 |
| 7. | hsa-miR-625* | 40.51 | GACUAUAGAACUUUCCCCCUCA | MIMAT0004808 |
| 8. | hsa-miR-505* | 33.39 | GGGAGCCAGGAAGUAUUGAUGU | MIMAT0004776 |
| 9. | hsa-miR-194 | 31.43 | UGUAACAGCAACUCCAUGUGGA | MIMAT0000460 |
| 10. | hsa-miR-1255B | 19.19 | CGGAUGAGCAAAGAAAGUGGUU | MIMAT0005945 |
| 11. | hsa-miR-362-3p | 14.54 | AACACACCUAUUCAAGGAUUCA | MIMAT0004683 |
| 12. | mmu-miR-451 | 8.37 | AAACCGUUACCAUUACUGAGUU | MIMAT0001631 |
| 13. | hsa-miR-20a | 4.19 | UAAAGUGCUUAUAGUGCAGGUAG | MIMAT0000075 |
| 14 | hsa-miR-199a-3p | 3.02 | ACAGUAGUCUGCACAUUGGUUA | MIMAT0004563 |
| 15 | hsa-miR-30d | 2.92 | UGUAAACAUCCCCGACUGGAAG | MIMAT0000245 |
| 16 | hsa-miR-328 | 2.56 | CUGGCCCUCUCUGCCCUUCCGU | MIMAT0000752 |
| 17 | hsa-miR-27b | 2.51 | UUCACAGUGGCUAAGUUCUGC | MIMAT0000419 |
| 18 | hsa-miR-195 | 2.46 | UAGCAGCACAGAAAUAUUGGC | MIMAT0000461 |
| 19 | hsa-miR-27a | 2.06 | UUCACAGUGGCUAAGUUCCGC | MIMAT0000084 |
| 20 | hsa-miR-361 | 2.69 | UUAUCAGAAUCUCCAGGGGUAC | MIMAT0000703 |
| 21 | hsa-miR-93 | 5.88 | ACUGCUGAGCUAGCACUUCCCG | MIMAT0004509 |
| 22 | hsa-miR-92a | 3.77 | UAUUGCACUUGUCCCGGCCUGU | MIMAT0000092 |

In Table 4, data was normalized using global normalization and was compared with healthy controls and orthopedic injury samples. Adjusted p value<0.05 calculated using Benjamin Hochberg algorithm.

**Table 5: MiRNAs altered in serum samples of sTBI.**

| S# | Micro RNA | Fold Change | Mature Sequence | Mirbase ID |
|---|---|---|---|---|
| 1 | hsa-miR-34a | 5128.61384 | UGGCAGUGUCUUAGCUGGUUGU | MIMAT0000255 |
| 2 | hsa-miR-486 | 281.6657 | UCCUGUACUGAGCUGCCCCGAG | MIMAT0002177 |
| 3 | hsa-miR-455 | 122.4616199 | UAUGUGCCUUUGGACUACAUCG | MIMAT0003150 |
| 4 | hsa-miR-624 | 114.5512941 | UAGUACCAGUACCUUGUGUUCA | MIMAT0003293 |
| 5 | hsa-miR-942 | 86.9048 | UCUUCUCUGUUUUGGCCAUGUG | MIMAT0004985 |
| 6 | hsa-miR-130b | 59.04301 | CAGUGCAAUGAUGAAAGGGCAU | MIMAT0000691 |
| 7 | hsa-miR-296 | 43.17099 | AGGGCCCCCCCUCAAUCCUGU | MIMAT0000690 |
| 8 | hsa-miR-505* | 36.61881 | GGGAGCCAGGAAGUAUUGAUGU | MIMAT0004776 |
| 9 | mmu-miR-491 | 34.9945167 | AGUGGGGAACCCUUCCAUGAGG | MIMAT0002807 |
| 10 | hsa-miR-151-5p | 29.7126 | UCGAGGAGCUCACAGUCUAGU | MIMAT0004697 |
| 11 | hsa-miR-579 | 18.64192 | UUCAUUUGGUAUAAACCGCGAUU | MIMAT0003244 |
| 12 | hsa-miR-339-3p | 13.99902 | UGAGCGCCUCGACGACAGAGCCG | MIMAT0004702 |
| 13 | hsa-miR-362-3p | 13.73953 | AACACACCUAUUCAAGGAUUCA | MIMAT0004683 |
| 14 | hsa-miR-19a | 5.525949 | UGUGCAAAUCUAUGCAAAACUGA | MIMAT0000073 |
| 15 | hsa-miR-9* | 4.742191 | AUAAAGCUAGAUAACCGAAAGU | MIMAT0000442 |
| 16 | hsa-miR-30d | 4.555606 | UGUAAACAUCCCCGACUGGAAG | MIMAT0000245 |
| 17 | hsa-miR-601 | 4.121515 | UGGUCUAGGAUUGUUGGAGGAG | MIMAT0003269 |
| 18 | hsa-miR-1291 | 3.716838 | UGGCCCUGACUGAAGACCAGCAGU | MIMAT0005881 |
| 19 | hsa-miR-195 | 3.515882 | UAGCAGCACAGAAAUAUUGGC | MIMAT0000461 |
| 20 | hsa-miR-660 | 2.841981 | UACCCAUUGCAUAUCGGAGUUG | MIMAT0003338 |
| 21 | hsa-miR-328 | 2.015088 | CUGGCCCUCUCUGCCCUUCCGU | MIMAT0000752 |
| 22 | hsa-miR-29c | 2.80347 | UAGCACCAUUUGAAAUCGGUUA | MIMAT0000681 |
| 23 | mmu-miR-451 | 2.569689 | AAACCGUUACCAUUACUGAGUU | MIMAT0001631 |
| 24 | hsa-miR-20a | 2.312593 | UAAAGUGCUUAUAGUGCAGGUAG | MIMAT0000075 |
| 25 | hsa-miR-27a | 1.813638 | UUCACAGUGGCUAAGUUCCGC | MIMAT0000084 |
| 26 | hsa-miR-92a | 2.57 | UAUUGCACUUGUCCCGGCCUGU | MIMAT0000092 |

In Table 5, data was normalized using global normalization and was compared with healthy controls and orthopedic injury samples. Adjusted p value<0.05 calculated using Benjamin Hochberg algorithm.

The analysis identified a novel signature of miRNAs whose expression was elevated in both sTBI and mTBI groups which were then selected for further biomarker analysis (Figures 2 and 3).

Table 6 shows one embodiment of the signature miRNA biomarkers used to identify mTBI and sTBI. The miRNA biomarkers as shown in Table 6 were present in samples from subjects with mTBI and sTBI, but not in samples from subjects with orthopedic injury.

**Table 6: miRNA biomarkers for TBI.**

| MiRNA | Mature Sequence | Mirbase ID |
|---|---|---|
| hsa-miR-151-5p | UCGAGGAGCUCACAGUCUAGU | MIMAT0004697 |
| hsa-miR-328 | CUGGCCCUCUCUGCCCUUCCGU | MIMAT0000752 |
| hsa-miR-486 | UCCUGUACUGAGCUGCCCCGAG | MIMAT0002177 |
| hsa-miR-362-3p | AACACACCUAUUCAAGGAUUCA | MIMAT0004683 |
| hsa-miR-942 | UCUUCUCUGUUUUGGCCAUGUG | MIMAT0004985 |
| hsa-miR-505* | GGGAGCCAGGAAGUAUUGAUGU | MIMAT0004776 |
| hsa-miR-195 | UAGCAGCACAGAAAUAUUGGC | MIMAT0000461 |
| hsa-miR-20a | UAAAGUGCUUAUAGUGCAGGUAG | MIMAT0000075 |
| hsa-miR-27a | UUCACAGUGGCUAAGUUCCGC | MIMAT0000084 |
| hsa-miR-30d | UGUAAACAUCCCCGACUGGAAG | MIMAT0000245 |
| mmu-miR-451 | AAACCGUUACCAUUACUGAGUU | MIMAT0001631 |
| has-miR-92a | UAUUGCACUUGUCCCGGCCUGU | MIMAT0000092 |

Functional pathway analysis of altered miRNAs and their association with TBI related gene targets was performed using Ingenuity Pathway Analysis (IPA) program (Ingenuity Systems Inc., Redwood City, CA). In IPA, there are currently 87 target molecules whose association has been linked with miRNA regulation in TBI. The eighty seven TBI related molecules were used to identify direct relation of the targets with certain candidate miRNAs shown in Table 6. The pathway explorer function of IPA was used to build putative pathways between TBI miRNA biomarker candidates and TBI related molecules. Thirty genes were identified as direct targets for TBI and nine miRNA candidates were identified as direct biomarkers, including miR-151-5p, miR-27a, miR-195, miR-328, miR-362-3p, miR-30d, miR-20a, miR-486 and miR-942. These genes were further analyzed by overlying them in the canonical pathway category. This analysis identified that most of the molecules predicted to be targeted by the miRNAs are involved in major TBI related canonical pathways such as erythropoietin signaling, G protein coupled receptor signaling, GABA receptor signaling and , neuropathic pain signaling in dorsal horn neurons. Overall, it was found that all the most of the miRNAs target important neurological pathways (Figure 3).

As discussed above, the eighty seven TBI related molecules that are available in the disease and function category were used, and any direct relation of these targets with the 10 candidate miRNAs were also identified. The pathway explorer function of IPA was used to build putative pathways between TBI miRNA biomarker candidates and TBI related molecules. This analysis identified 30 genes as direct targets for the 8 miRNA candidate miR-151-5p, miR-195, miR-328-3p, miR-362-3p, miR-30d, miR-20a, miR-486 and miR-92a. MiR-505* and miR-451 were not predicted to target any of the target molecules for TBI in IPA. These genes were further analyzed by overlying them in the canonical pathway category. This analysis identified that most of the molecules predicted to be targeted by the miRNAs are involved in major TBI related canonical pathways such as erythropoietin signaling, G protein coupled receptor signaling, GABA receptor signaling, and neuropathic pain signaling in dorsal horn neurons. Specifically, miR-328 was predicted to regulate erythropoietin and erythropoietin receptor which are important mediators of erythropoietin signaling. MiR-486, miR-27a and miR-195 targeted molecules involved in glutamate receptor signaling and GABA receptor signaling. MiR-151-5p and miR-362-3p target molecule SCN4A which is shown to be responsible for generation and propagation of neurons. MiR-30d was also predicted to target adrenoceptors and GABA receptor signaling. Overall, it was found that all the most of the miRNAs target important neurological pathways (Figure 4).

To validate the findings of the methods of detecting miRNA levels using TaqMan Low Density Human MicroRNA array cards (TLDA) platform, specific miRNA PCR was performed for selected miRNAs: miR-195, miR-505*, miR-151-5p, miR-328, miR-362-3p, miR-486 and miR-942. To perform the specific miRNA PCR assays, an endogenous control was required. For specific assays, an endogenous control was identified by selecting the miRNA with the least standard deviation in the delta Ct values obtained after global normalization. MiR-202 was identified and selected as endogenous control for all the specific PCR validation experiments. RNA was again isolated from the serum samples and assays were performed without pre-amplification of cDNA. The validation showed significant upregulation of the miRNAs in both mTBI and sTBI groups as observed previously in the miRNA profiling result (Figure 5). The expression value of miR-151-5p, however, was not significantly upregulated in mTBI injury though its expression was upregulated in sTBI (Figure 5). The results demonstrate that all the selected miRNAs were significantly upregulated after TBI.

To validate the presence of miRNAs observed in serum studies, a complete miRNA profiling was performed using CSF samples from sTBI patients (n=8) and control CSF samples (n=6). MiR-202 was selected as the endogenous control for the specific PCR assays in the CSF samples. The conventional miRNA assay was modified by adding an additional pre-amplified the product using the real time primers, which does not introduce additional bias since only one primer is used for pre-amplification reaction. The real time data for miR-151-5p, miR-328, miR-362-3p, miR-486 and miR-942 were normalized using miR-202. MiR-202 was found stable in the CSF samples with a mean Ct value of 26.2 and 25.8 in injury and control samples respectively. Normalization with miR-202 showed a significant upregulation of miR-328, miR-362-3p and miR-486 (Figure 6). Increase in miR-151-5p was also observed.

Additional miRNA assays for candidate miRNAs identified in serum as biomarker candidates in both MMTBI and STBI groups. The conventional miRNA assay methodology was modified and an additional pre-amplification step was added in the analysis. This pre-amplification does not introduce additional bias since only one primer is used for pre-amplification reaction. The real time data for miR-151-5p, miR-195, miR-20a, miR-30d, miR-328, miR-362-3p, miR-451, miR-486, miR-505* and miR-92a was normalized using miR-202. MiR-202 was found extremely stable in the CSF samples with a mean Ct value of 26.2 and 25.8 in injury and control samples respectively. Normalization with miR-202 showed a significant upregulation of miR-328, miR-362-3p, miR-451 and miR-486 (Figure 7). For miR-505* and miR-195, although the mean fold upregulation was more than 10 fold, however it was only observed in 50-60% of the samples whereas in the remaining samples it was not detected, hence these failed the statistical test. Similar observation was also found for miR-20a. An increase in miR-151-5p was observed, but it was not significant due to sample outliers. No significant upregulation in the level of miR-30d was observed between control and injury groups.

The miRNA data was analyzed with the delta Ct data from the real time PCR data of the TBI and trauma control groups to identify a correlation of miRNAs with CT lesions. The comparison between these groups was performed using the delta Ct values because of the absence of absolute fold change. A comparison of level of miRNA was performed in 2 groups of human subjects comprised of (1) subjects (TBI and all controls) without any lesions on head CT (n=19); and (2) TBI subjects with lesions on head CT (n=12). The assumption was made that all normal and trauma controls had negative CT scans. There were significant differences between the two groups for all but two of the selected miRNA: miR-195 (p≤0.001); miR-30d (p≤0.001); miR-451 (p≤0.011); miR-328 (p≤0.101); miR-92a (p≤0.001); miR-486 (p≤0.006); miR-505 (p≤0.008); and miR-362 (p≤0.035); miR-151 (p≤0.065); and miR-20a (p≤0.012) (Figure 8).

Receiver operator characteristic (ROC) curve was generated to calculate the area under the curve (AUC) to identify the accuracy of the miRNAs in diagnosing TBI. The analysis identified the AUC values as miR-195 (0.81, p value < 0.003), miR-30d (0.75, p value <0.016), miR-451 (0.82, p value <0.002), miR-328 (0.73, p value < 0.030), miR-92a (0.86, p value <0.001), miR-486 (0.81, p value <0.003), miR-505 (0.82, p value <0.002), miR-362 (0.79, p value <0.006), miR-151 (0.66, p value < 0.123), miR-20a (0.78, 0.007). All miRNAs except for miR-151 showed good diagnostic accuracy (Figure 9).

## Claims

1. A method of diagnosing severe traumatic brain injury (TBI) in a human subject with a head injury, the method comprising:
a) determining level(s) of one or more micro RNAs (miRNAs) in a serum biological sample taken from the subject; and
b) comparing the determined level(s) of the one or more miRNAs against level(s) of the same one or more miRNAs from a control subject determined not to be suffering from TBI or from the same subject at an earlier timepoint;
wherein the one or more miRNAs comprise hsa-miR-151-5p; and
wherein a fold change of a level of hsa-miR-151-5p above 5 compared to the control level determined in step b) is indicative that the subject is suffering from severe TBI.

2. The method of claim 1, wherein the head injury is a closed head injury (CHI) or a blast-induced traumatic brain injury (bTBI).

3. The method of claims 1 or 2, wherein the biological sample is taken from the subject less than one day after a head injury.

4. The method of any of claims 1-3, wherein the biological sample is taken from the subject between two and eight days after a head injury.

5. The method of any of claims 1-4, wherein the level(s) of one or more specific micro RNAs are determined by a real time PCR.

6. The method of any of claims 1-5, wherein the amount of an miRNA is compared to the amount of hsa-miR-202 to determine the level of that miRNA.

## Patentansprüche

1. Verfahren zur Diagnose eines schweren Schädel-Hirn-Traumas (SHT) bei einer menschlichen Person mit einer Kopfverletzung, wobei das Verfahren umfasst:
a) Bestimmen von Pegel(n) einer oder mehrerer Mikro-RNAs (miRNAs) in einer biologischen Serumprobe, die der Person entnommen wurde; und
b) Vergleichen des/der bestimmten Pegel(s) der einen oder mehreren miRNAs mit Pegel(n) der gleichen einen oder mehreren miRNAs einer Kontrollperson, bei der festgestellt wurde, dass sie nicht an SHT leidet, oder von der gleichen Person zu einem früheren Zeitpunkt;
wobei die eine oder mehrere miRNAs hsa-miR-151-5p umfassen; und
wobei eine Änderung eines Pegels von hsa-miR-151-5p über das 5-Fache im Vergleich zu dem in Schritt b) bestimmten Kontrollpegel darauf hinweist, dass die Person an schwerem SHT leidet.

2. Verfahren nach Anspruch 1, wobei die Kopfverletzung eine geschlossene Kopfverletzung (CHI) oder ein durch Explosionen induziertes Schädel-Hirn-Trauma (bSHT) ist.

3. Verfahren nach Anspruch 1 oder 2, wobei die biologische Probe der Person weniger als einen Tag nach einer Kopfverletzung entnommen wird.

4. Verfahren nach Anspruch 1-3, wobei die biologische Probe der Person zwischen zwei und acht Tagen nach einer Kopfverletzung entnommen wird.

5. Verfahren nach Anspruch 1-4, wobei der (die) Pegel einer oder mehrerer spezifischer Mikro-RNAs durch eine Echtzeit-PCR bestimmt wird (werden).

6. Verfahren nach Anspruch 1-5, wobei die Menge einer miRNA mit der Menge von hsa-miR-202 verglichen wird, um die Menge dieser miRNA zu bestimmen.

## Revendications

1. Procédé de diagnostic d'une lésion cérébrale traumatique (TBI) grave chez un sujet humain souffrant d'un traumatisme crânien, le procédé comprenant :
a) la détermination du(des) taux d'un ou plusieurs micro-ARN (miARN) dans un échantillon biologique sérique prélevé sur le sujet ; et
b) la comparaison du(des) taux déterminé(s) des un ou plusieurs miARN au(x) taux des un ou plusieurs miARN identiques provenant d'un sujet témoin déterminé comme ne souffrant pas de TBI ou du même sujet à un moment antérieur ;
dans lequel les un ou plusieurs miARN comprennent hsa-miR-151-5p ; et
dans lequel un facteur de changement d'un taux de hsa-miR-151 -5p supérieur à 5 par rapport au taux témoin déterminé à l'étape b) indique que le sujet souffre d'une TBI grave.

2. Procédé selon la revendication 1, dans lequel le traumatisme crânien est un traumatisme crânien fermé (CHI) ou une lésion cérébrale traumatique induite par explosion (bTBI).

3. Procédé selon les revendications 1 ou 2, dans lequel l'échantillon biologique est prélevé sur le sujet moins d'un jour après un traumatisme crânien.

4. Procédé selon l'une quelconque des revendications 1 à 3, dans lequel l'échantillon biologique est prélevé sur le sujet entre deux et huit jours après un traumatisme crânien.

5. Procédé selon l'une quelconque des revendications 1 à 4, dans lequel le(s) taux d'un ou plusieurs micro-ARN spécifiques sont déterminés par une PCR en temps réel.

6. Procédé selon l'une quelconque des revendications 1 à 5, dans lequel la quantité d'un miARN est comparée à la quantité de hsa-miR-202 pour déterminer le taux de ce miARN.
